# EUROPÄISCHE PATENTSCHRIFT

(11) **EP 0 797 558 B1**
(45) Veröffentlichungstag und Bekanntmachung des Hinweises auf die Patenterteilung: **14.11.2001**
(21) Anmeldenummer: 95905584.9
(22) Anmeldetag: 23.12.1994
(51) Int. Cl.: C04B 41/90, C04B 37/02, H01R 43/02, H01R 4/02

(54) **ELEKTRISCH LEITENDE VERBINDUNG**
ELECTRICALLY CONDUCTIVE CONNECTION
LIAISON ELECTRIQUEMENT CONDUCTRICE

(30) Priorität: 05.01.1994 DE 4400220
(43) Veröffentlichungstag der Anmeldung: 01.10.1997
(73) Patentinhaber: Heraeus Electro-Nite International N.V., 3530 Houthalen (BE)
(72) Erfinder: SCHÖNAUER, Ulrich, D-76131 Karlsruhe (DE); TAFFERNER, Michael, D-76316 Malsch (DE); FISCHER, Hagen, D-76137 Karlsruhe (DE)
(74) Vertreter: Kühn, Hans-Christian
(86) Internationale Anmeldenummer: EP9404297
(87) Internationale Veröffentlichungsnummer: WO9518777

(56) Entgegenhaltungen:
- EP-A- 0 113 895
- EP-A- 0 277 645
- DE-B- 1 022 957
- US-A- 4 652 727

## Beschreibung

Die Erfindung betrifft eine elektrisch leitende Verbindung zwischen einem metallischen Anschlußstück und einer auf einem Keramiksubstrat, welches vorzugsweise Glas und/oder Glaskeramik in geringen Mengen aufweist, durch eine Sinterverbindung aufgebrachten, zumindest ein Metall aufweisenden Metallschicht, auf die das Anschlußstück geschweißt ist.

Man hat schon daran gedacht, solche vorstehend angegebenen Verbindungen herzustellen. Eine derartige Verbindung ist aus DE-B-10 22 957 bekannt. Hier ist ein Metall-Keramik-Verbundkörper beschrieben, der aus dünnen Schichten gebildet und für die Herstellung von Kondensatoren geeignet ist. EP-A-0 113 895 offenbart ein Verfahren zum Laserlöten von flexiblen Verdrahtungen auf Glassubstraten. Bei dem Verfahren werden Drähte mittels eines Niederhalters auf ein Glassubstrat gedrückt. Der Niederhalter ist lichtdurchlässig, so daß der Laserstrahl das Lot aufschmelzen kann.

Solche im Labor hergestellten Verbindungen weisen jedoch diverse Nachteile auf. So ist die Verbindung zwischen dem Keramiksubstrat und dem Metall aufgrund der sehr unterschiedlichen Eigenschaften der Werkstoffe der zu verbindenden Teile, insbesondere der unterschiedlichen Ausdehnungskoeffizienten und chemischen Zusammensetzungen kritisch. Die Haftung an der Grenzfläche zwischen dem Keramiksubstrat einerseits und der aufgesinterten Metallschicht andererseits ist äußerst gering, weil das Metall und die Keramik nur einen geringen Verbund bilden, andererseits die Adhäsionskräfte sehr gering sind. Insbesondere beim Einsatz im Hochtemperaturbereich, insbesondere wenn mechanische Belastungen hinzukommen, kann eine hinreichend zuverlässige Verbindung nicht gewährleistet werden. Hinzu kommt, daß durch die Schweißung, wenn sie nicht auf einer hinreichend dicken Metallschicht und/oder einem metallischen Anschlußstück erfolgt, eine Zerstörung der Metallschicht bzw. der darunter liegenden Schichten, z.B. des Keramiksubstrates bewirkt wird, wobei dann die auf dem Keramiksubstrat aufgebrachte elektrische und /oder mechanische Anordnung nicht mehr funktionsfähig ist. Insgesamt wird also bei der Herstellung der elektrisch leitenden Verbindung entweder ein hoher Ausschuß erzeugt, oder aber die elektrische Verbindung ist nicht hinreichend mechanisch stabil und temperaturfest.

Der Erfindung liegt die Aufgabe zugrunde, einen Verbund sowie ein Verfahren zu dessen Herstellung vorzuschlagen, welche bessere und dauerhafte Verbindungen zuläßt.

Diese Aufgabe wird erfindungsgemäß durch die Gegenstände von Anspruch 1 und 12 gelöst. Die Verwendung des Verbundes ist in Anspruch 11 beansprucht. Vorteilhafte Ausführungsformen des Verbundes und des Verfahrens sind in der Ansprüchen 2 bis 10 und 13 bis 15 beansprucht. Nach der erfindungsgemäßen Lehre wird also die 20 bis 100 µm dicke Metallschicht nicht direkt auf das Keramiksubstrat aufgebracht, sondern zwischen diesen beiden Schichten wird eine 5 bis 15 µm dicke Haftvermittlungsschicht aufgebracht, die Glas und/oder Glaskeramik sowie Metallteilchen als Flitter, in Pulverform oder dergleichen enthält und auf das Keramiksubstrat , vorzugsweise vermittels einer Schmelzverbindung aufgeschmolzen wird. Hierbei findet eine Schmelzverbindung zwischen dem in der Haftvermittlungsschicht enthalten , Glas und/oder der Glaskeramik einerseits und den Glasanteilen sowie dem Keramiksubstrat oder diesem alleine andererseits statt. Infolgedessen ergibt sich eine kraftschlüssige Verbindung zwischen dem Keramiksubstrat und der Haftvermittlungsschicht. Zugleich verbinden sich die Metallteilchen der Haftvermittlungsschicht mit den aufschmelzenden bzw. erweichenden Glasbestandteilen. Durch dieses Umfließen wird ein Formschluß der verschiedenen, kornförmigen Bestandteile und damit eine innige Verbindung zwischen den Teilchen der Haftvermittlungsschicht erzielt. Auf diese Haftvermittlungsschicht wird dann die reine Metallschicht z.B. in Dickschichttechnik aufgebracht, und durch Sintern mit ihr verbunden. Bei dieser Sinterverbindung wird eine intermetallische Verbindung der metallischen Teile der Metallschicht mit den Metallteilchen in der Haftvermittlungsschicht bewirkt. An dieser wird durch eine Sinterverbindung ein mechanisch fester Verbund erzielt. Auf die Metallschicht wird dann ein metallisches Anschlußstück als Kontaktfeder, Kontaktklipp oder Metallfolie aufgelegt und mit ganz besonderem Vorteil, weil sehr schnell und berührungslos, mittels Laserschweißung elektrisch gut leitend einerseits, sowie mechanisch äußerst stabil andererseits mit der Metallschicht verbunden. Bei Einsatz entsprechender hochtemperaturbeständiger Metalle, z.B. Edelmetalle wie Platin oder deren Legierungen ergibt sich eine hochtemperaturfeste und elektrisch gutleitende sowie mechanisch äußerst robuste Verbindung. Sie eignet sich insbesondere in stark oxidierenden sowie korrosiven Umgebungen bei hoher Temperatur, wie z.B. im heißen und pulsierenden Abgas von Verbrennungsmotoren als Verbindungstechnik bei Gassensoren z.B. Lambda-Sonden.

Überraschenderweise hat sich ferner herausgestellt, daß die elektrisch leitende Verbindung mechanisch so stabil ist, daß sie zugleich als mechanische Halterung dienen kann. Insgesamt ergibt sich also eine feste und dauerhafte, elektrisch zuverlässige Verbindung, die sich einfach und kostengünstig mit hoher Reproduzierbarkeit und Prozeßsicherheit beim Handling und bei der Herstellung darstellen läßt. Außerdem ergibt sich eine kostengünstige Integrierbarkeit in dem Prozeß- und Montageablauf, in welchem bereits Verbindungen zwischen zwei Metallen mittels Laserschweißen hergestellt werden. Außerdem ergibt sich beim Laserschweißen der Vorteil einer hohen Standzeit. Bei der Wahl geeigneter Materialien, z.B. Edelmetallen ist der Einsatz bei hohen Temperaturen und oxidierender sowie korrosiver Umgebung gut möglich, ohne daß die mechanische oder elektrische Verbindung hierunter leidet. Von besonderem Vorteil gestaltet sich die Verbindung, wenn das Metall der Metallschicht und jenes der Metallteilchen der Haftvermittlungsschicht identisch ist, gegenüber nur metallurgisch ähnlichen bzw. verwandten Metallen oder deren Legierungen.

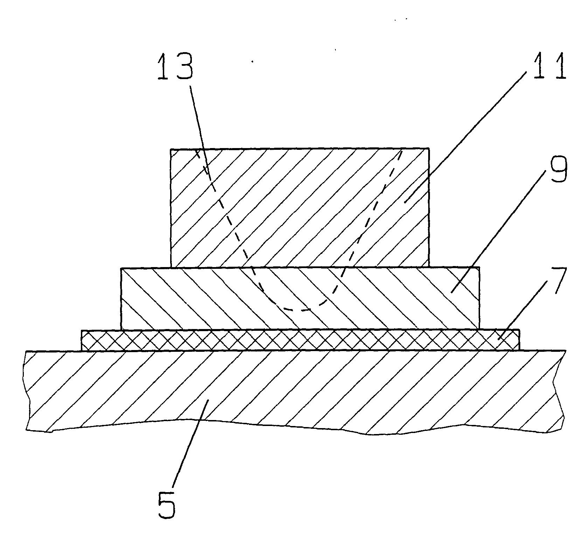

Zweckmäßige Ausgestaltungen und Weiterbildungen der Erfindung sind in den Unteransprüchen gekennzeichnet. Ein Ausführungsbeispiel der Erfindung wird nachfolgend unter Bezugnahme auf die Zeichnung näher erläutert, welche einen schematischen Querschnitt durch eine Verbindung zeigt.

Hierbei ist das Keramiksubstrat mit 5 bezeichnet und weist überwiegend Al₂O₃ mit einem geringen Anteil von Glas und/oder Glaskeramik von ca. 4 % auf. Darüber ist die erfindungsgemäße Haftvermittlungsschicht 7 mittels einer Schmelzverbindung aufgebracht. Die Haftvermittlungsschicht enthält Metallteilchen in Flitter- und/oder Pulver- oder dergleichen Form sowie Glas und weist (am fertigen Endprodukt mit allen anderen Schichten) eine Dicke der Haftvermittlungsschicht von 7 bis 8 *µ*m auf.

Über der Haftvermittlungsschicht 7 ist die eigentliche Metallschicht 9 aufgesintert, welche im Ausgangszustand Metall, vorzugsweise Edelmetall in Teilchenform, z.B. als Pulver, Flitter oder dergleichen enthält. Auf diese Metallschicht 9 wird dann ein metallisches Anschlußstück 11, beispielsweise ein Klipp oder dergleichen aufgelegt und dann mittels eines Laserschweißpunktes mit der Metallschicht 9 verbunden. Der hierbei aufgeschmolzene Bereich ist gestrichelt mit 13 bezeichnet und zeigt, daß nicht nur das größer als 100 *µ*m dicke elektrische Anschlußstück, sondern auch die etwa 40 bis 60 *µ*m dicke Metallschicht (am fertigen Endprodukt mit allen anderen Schichten) bereichsweise etwas aufgeschmolzen wird, wodurch sich die innige mechanische sowie elektrisch gut leitende Verbindung zwischen dem Anschlußstück 11 und der zu einer elektrischen Anordnung auf dem Keramiksubstrat 5 führenden Metallschicht 9 bildet. Die Laserschweißung hat überdies den Vorteil, daß extrem kleine Punkte sehr genau und mit hoher Reproduzierbarkeit geschweißt werden können.

## Patentansprüche

1. Verbund, enthaltend ein Keramik substrat (5), eine Haftvermittlungsschicht (7), eine Metallschicht (9) und ein Anschlußstück (11), wobei eine elektrisch leitende Verbindung zwischen dem metallischen Anschlußstück (11) und der auf dem Keramiksubstrat (5) durch eine Sinter- und/oder Schmelzverbindung aufgebrachten, zumindest ein Metall aufweisenden Metallschicht (9), auf der das Anschlußstück (11) geschweißt ist, vorgesehen ist, wobei auf das Keramiksubstrat (5) die Glas und/oder Glaskeramik sowie Metallteilchen aufweisende Haftvermittlungsschicht (7) und auf diese die Metallschicht (9) aufgebracht ist, wobei die Haftvermittlungsschicht (7) eine Dicke von 5 bis 15 µm und die Metallschicht (9) eine Dicke von 20 bis 100 µm - jeweils bezogen auf die fertige elektrisch leitende Verbindung - aufweisen, und wobei das Anschlußstück (11) mittels einer Schweißverbindung mit der Metallschicht verbunden ist.

2. Verbund nach Anspruch 1, **dadurch gekennzeichnet, daß** das Anschlußstück (11) mittels einer Laserschweißverbindung mit der Metallschicht (9) verbunden ist.

3. Verbund nach Anspruch 1 oder 2, **dadurch gekennzeichnet, daß** die Haftvermittlungsschicht (7) eine Dicke von 7 bis 12 µm aufweist.

4. Verbund nach Anspruch 1, 2 oder 3, **dadurch gekennzeichnet, daß** die Metallschicht (9) eine Dicke von 25 bis 60 µm aufweist.

5. Verbund nach einem der Ansprüche 1 bis 4, **dadurch gekennzeichnet, daß** die Haftvermittlungsschicht (7) in ihrem Ausgangszustand Metallteilchen als pulverförmiges Metall und/oder Metallflitter aufweist.

6. Verbund nach einem der Ansprüche 1 bis 5, **dadurch gekennzeichnet, daß** das metallische Anschlußstück (11) eine Folie, ein Plättchen, ein Draht oder ein Klipp ist.

7. Verbund nach Anspruch 6, **dadurch gekennzeichnet, daß** die Dicke des metallischen Anschlußstücks (11) zwischen 10 und 300 µm liegt.

8. Verbund nach einem der Ansprüche 1 bis 7, **dadurch gekennzeichnet, daß** die Haftvermittlungsschicht (7) und/oder die Metallschicht (9) zumindest ein Edelmetall, vorzugsweise Platin aufweist.

9. Verbund nach einem der Ansprüche 1 bis 8, **dadurch gekennzeichnet, daß** das Anschlußstück (11) aus einem korrosionsbeständigen Werkstoff besteht.

10. Verbund nach einem der Ansprüche 1 bis 9, **dadurch gekennzeichnet, daß** das Keramiksubstrat (5) Glas und/oder Glaskeramik in geringen Mengen aufweist.

11. Verwendung eines Verbundes nach einem der Ansprüche 1 bis 10 in einem heißen Abgas von Verbrennungsmotoren als elektrischer Anschluß und/oder mechanischer Befestigung für ein Keramiksubstrat (5) eines Sensors, vorzugsweise Lambda-Sonde.

12. Verfahren zur Herstellung einer elektrisch leitenden Verbindung zwischen einem metallischen Anschlußstück (11) und einer auf einem Keramiksubstrat (5) durch eine Sinterverbindung aufgebrachten, zumindest ein Metall aufweisenden Metallschicht (9), auf die das Anschlußstück (11) geschweißt ist, wobei auf das Keramiksubstrat (5) eine Glas und/oder Glaskeramik sowie Metallteilchen aufweisende Haftvermittlungsschicht (7) in Dickschichttechnik aufgebracht und danach aufgesintert und/oder aufgeschmolzen wird, wobei auf die Haftvermittlungsschicht (7) die Metallschicht (9) in Dickschichttechnik aufgebracht und aufgesintert wird, wobei die Haftvermittlungsschicht (7) eine Dicke von 5 bis 15 µm und die Metallschicht (9) eine Dicke von 20 bis 100 um - jeweils bezogen auf die fertige elektrisch leitende Verbindung - aufweisen, und wobei auf diese das Anschlußstück (11) vorzugsweise durch Laserschweißung geschweißt wird.

13. Verfahren nach Anspruch 12, **dadurch gekennzeichnet, daß** das Keramiksubstrat (5) Glas und/oder Glaskeramik in geringen Mengen aufweist.

14. Verfahren nach Anspruch 12 oder 13, **dadurch gekennzeichnet, daß** die Metallschicht (9) auf die Haftvermittlungsschicht (7) aufgebracht wird und danach beide Schichten gemeinsam gesintert werden.

15. Verfahren nach einem der Ansprüche 12 bis 14, **dadurch gekennzeichnet, daß** die Haftvermittlungsschicht (7) und/oder die Metallschicht (9) als Dickschicht in Sieb- und/oder Schablonendrucktechnik aufgebracht werden.

## Claims

1. Composite comprising a ceramic substrate (5), an adhesion-promoting layer (7), a metal layer (9) and a connecting piece (11), an electrically conducting connection being provided between the metallic connecting piece (11) and the metal layer (9) which is connected to the ceramic substrate (5) by sintering and/or fusion and comprises at least one metal and to which the connecting piece (11) is welded, the glass and/or glass ceramic as well as the adhesion-promoting layer (7) comprising metal particles being applied to the ceramic substrate (5) and the metal layer (9) being applied to said adhesion-promoting layer, the adhesion-promoting layer (7) having a thickness of from 5 to 15 µm and the metal layer (9) having a thickness of from 20 to 100 µm, based in each case on the finished electrically conducting connection, and the connection piece (11) being connected to the metal layer by means of a weld joint.

2. Composite according to Claim 1, **characterized in that** the connecting piece (11) is connected to the metal layer (9) by means of a laser weld joint.

3. Composite according to Claim 1 or 2, **characterized in that** the adhesion-promoting layer (7) has a thickness of from 7 to 12 µm.

4. Composite according to Claim 1, 2 or 3, **characterized in that** the metal layer (9) has a thickness of from 25 to 60 µm.

5. Composite according to any of Claims 1 to 4, **characterized in that** the adhesion-promoting layer (7) in its initial state comprises metal particles as pulverulent metal and/or metal spangles.

6. Composite according to any of Claims 1 to 5, **characterized in that** the metallic connecting piece (11) is a foil, a lamella, a wire or a clip.

7. Composite according to Claim 6, **characterized in that** the thickness of the metallic connecting piece (11) is between 10 and 300 µm.

8. Composite according to any of Claims 1 to 7, **characterized in that** the adhesion-promoting layer (7) and/or the metal layer (9) comprises at least one noble metal, preferably platinum.

9. Composite according to any of Claims 1 to 8, **characterized in that** the connecting piece (11) consists of a corrosion-resistant material.

10. Composite according to any of Claims 1 to 9, **characterized in that** the ceramic substrate (5) comprises glass and/or glass ceramic in small amounts.

11. Use of a composite according to any of Claims 1 to 10 in a hot exhaust gas from internal combustion engines as an electrical connection and/or mechanical fastening for a ceramic substrate (5) of a sensor, preferably a lambda probe.

12. Process for the production of an electrically conducting connection between a metallic connecting piece (11) and a metal layer (9) which is connected to a ceramic substrate (5) by sintering and comprises at least one metal and to which the connecting piece (11) is welded, a glass and/or glass ceramic as well as an adhesion-promoting layer (7) comprising metal particles being applied to the ceramic substrate (5) by the thick-film technique and then being sintered on and/or fused on, the metal layer (9) being applied to the adhesion-promoting layer (7) by the thick-film technique and being sintered on, the adhesion-promoting layer (7) having a thickness of from 5 to 15 µm and the metal layer (9) having a thickness of from 20 to 100 µm, based in each case on the finished electrically conducting connection, and the connecting piece (11) being welded thereon, preferably by laser welding.

13. Process according to Claim 12, **characterized in that** the ceramic substrate (5) comprises glass and/or glass ceramic in small amounts.

14. Process according to Claim 12 or 13, **characterized in that** the metal layer (9) is applied to the adhesion-promoting layer (7) and the two layers are then sintered together.

15. Process according to any of Claims 12 to 14, **characterized in that** the adhesion-promoting layer (7) and/or the metal layer (9) are applied as a thick film by the screen printing and/or stencil printing technique.

## Revendications

1. Composite contenant un substrat céramique (5), une couche promouvant l'adhérence (7), une couche métallique (9) et une pièce de raccordement (11), où il est prévu une liaison électriquement conductrice entre la pièce de raccordement métallique (11) et la couche métallique (9) comportant au moins un métal, sur laquelle est soudée la pièce de raccordement (11) et qui est appliquée sur le substrat céramique (5) par une liaison par frittage etlou par fusion, où la couche promouvant l'adhérence (7) comportant un verre et/ou une vitrocéramique ainsi que des particules métalliques est appliquée sur le substrat céramique (5) et la couche métallique (9) est appliquée sur la précédente, où la couche promouvant l'adhérence (7) a une épaisseur de 5 à 15 µm et la couche métallique (9) a une épaisseur de 20 à 100 µm, dans chaque cas par rapport à la liaison électriquement conductrice terminée, et où la pièce de raccordement (11) est liée à la couche métallique au moyen d'une liaison par soudage.

2. Composite selon la revendication 1, **caractérisé en ce que** la pièce de raccordement (11) est liée à la couche métallique (9) au moyen d'une liaison par soudage à laser.

3. Composite selon la revendication 1 ou 2, **caractérisé en ce que** la couche promouvant l'adhérence (7) a une épaisseur de 7 à 12 µm.

4. Composite selon la revendication 1, 2 ou 3, **caractérisé en ce que** la couche métallique (9) présente une épaisseur de 25 à 60 µm.

5. Composite selon l'une des revendications 1 à 4, **caractérisé en ce que** la couche promouvant l'adhérence (7) comporte dans son état initial des particules métalliques sous forme de métal pulvérulent et/ou de paillettes métalliques.

6. Composite selon l'une des revendications 1 à 5, **caractérisé en ce que** la pièce de raccordement métallique (11) est une feuille, une plaquette, un fil ou une attache.

7. Composite selon la revendication 6, **caractérisé en ce que** l'épaisseur de la pièce de raccordement métallique (11) est située entre 10 et 300 µm.

8. Composite selon l'une des revendications 1 à 7, **caractérisé en ce que** la couche promouvant l'adhérence (7) et/ou la couche métallique (9) comportent au moins un métal noble, de préférence le platine.

9. Composite selon l'une des revendications 1 à 8, **caractérisé en ce que** la pièce de raccordement (11) consiste en un matériau résistant à la corrosion.

10. Composite selon l'une des revendications 1 à 9, **caractérisé en ce que** le substrat céramique (5) comporte un verre et/ou une vitrocéramique en faibles quantités.

11. Utilisation d'un composite selon l'une des revendications 1 à 10 dans un gaz d'échappement chaud de moteurs à combustion interne sous forme de raccordement électrique et/ou de fixation mécanique pour un substrat céramique (5) d'un capteur, de préférence d'une sonde lambda.

12. Procédé de fabrication d'une liaison électriquement conductrice entre une pièce de raccordement métallique (11) et une couche métallique (9) comportant au moins un métal, sur laquelle est soudée la pièce de raccordement (11) et qui est appliquée sur un substrat céramique (5) par une liaison par frittage, où sur le substrat céramique (5) est appliquée par la technique des couches épaisses une couche promouvant l'adhérence (7) comportant un verre et/ou une vitrocéramique ainsi que des particules métalliques qui est ensuite frittée et/ou fondue, où la couche métallique (9) est appliquée et frittée par la technique des couches épaisses sur la couche promouvant l'adhérence (7), où la couche promouvant l'adhérence (7) a une épaisseur de 5 à 15 µm et la couche métallique (9) a une épaisseur de 20 à 100 µm, dans chaque cas par rapport à la liaison électriquement conductrice terminée, et où la pièce de raccordement (11) est soudée sur celle-ci de préférence par soudage à laser.

13. Procédé selon la revendication 12, **caractérisé en ce que** le substrat céramique (5) comporte un verre et/ou une vitrocéramique en faibles quantités.

14. Procédé selon la revendication 12 ou 13, **caractérisé en ce que** la couche métallique (9) est appliquée sur la couche promouvant l'adhérence (7), après quoi les deux couches sont frittées ensemble.

15. Procédé selon l'une des revendications 12 à 14, **caractérisé en ce que** la couche promouvant l'adhérence (7) et/ou la couche métallique (9) sont appliquées sous forme de couche épaisse par la technique de sérigraphie et/ou de pochoir.
